Europäisches Patentamt

European Patent Office

Office européen des brevets

(11) Numéro de publication : **0 456 552 B1**

(12) # FASCICULE DE BREVET EUROPEEN

(45) Date de publication du fascicule du brevet :
**19.01.94 Bulletin 94/03**

(51) Int. Cl.$^5$ : **C07C 17/20, C07C 19/08**

(21) Numéro de dépôt : **91401136.6**

(22) Date de dépôt : **29.04.91**

(54) **Procédé de fabrication du 1,1,1,2-tétrafluoro-chloroéthane et du pentafluoroéthane.**

(30) Priorité : **11.05.90 FR 9005908**

(43) Date de publication de la demande :
**13.11.91 Bulletin 91/46**

(45) Mention de la délivrance du brevet :
**19.01.94 Bulletin 94/03**

(84) Etats contractants désignés :
**AT BE CH DE DK ES FR GB GR IT LI LU NL SE**

(56) Documents cités :
**EP-A- 0 313 061
EP-A- 0 349 298
DE-A- 1 568 614
GB-A- 1 206 909
US-A- 2 110 369
PATENT ABSTRACTS OF JAPAN, vol. 9, no. 91
(C-277)(1814), 19 avril 1985, & JP-A-59 225131
PATENT ABSTRACTS OF JAPAN, vol. 5, no. 75
(C-55)(747), 19 mai 1981, & JP-A-56 24050**

(73) Titulaire : **ELF ATOCHEM S.A.
4 & 8, Cours Michelet La Défense 10
F-92800 Puteaux (FR)**

(72) Inventeur : **Cheminal, Bernard
"La Rivière", Orliénas
F-69530 Brignais (FR)**
Inventeur : **Lacroix, Eric
52 Rue Laennec
F-69008 Lyon (FR)**
Inventeur : **Lantz, André
Domaine de la Hêtraie
F-69390 Vernaison (FR)**

(74) Mandataire : **Leboulenger, Jean et al
ATOCHEM Département Propriété Industrielle
F-92091 Paris la Défense 10 Cédex 42 (FR)**

# EP 0 456 552 B1

## Description

La présente invention concerne un procédé continu de fabrication du 1,1,1,2-tétrafluoro-chloroéthane (F124) et du pentafluoroéthane (F125) et a plus particulièrement pour objet la fabrication de ces deux composés par fluoration en phase gazeuse du 1,1,1-trifluoro-dichloroéthane (F123) au moyen d'acide fluorhydrique en présence d'un catalyseur.

Les composés F124 et F125 pouvant être utilisés comme substituts des perchlorofluorocarbures (CFC) dans le domaine des aérosols (agents propulseurs) et dans celui de la réfrigération, on recherche actuellement des procédés performants pour leur production industrielle.

Dans le brevet US 4 766 260 est décrit un procédé de synthèse des composés F123 et F124 par hydrofluoration d'oléfines perhalogénées en phase gazeuse, l'objectif étant de minimiser la formation de F125. L'exemple 13 (colonne 6) décrit la fluoration du tétrachloroéthylène avec un catalyseur $CrCl_3/Al_2O_3$ ; malgré une température de 350°C, un long temps de contact (60 secondes) et un rapport molaire $HF/C_2Cl_4$ élevé (6/1), les sélectivités en F124 et F125 sont faibles (33,3 % et 7,2 % respectivement).

L'emploi d'un catalyseur à base de chrome III ($CrCl_3$) supporté sur charbon pour la fluoration catalytique en phase gazeuse d'oléfines halogénées fait l'objet de la demande de brevet japonaise publiée sous le n° 48-72105/73 dont l'exemple 4 décrit la fluoration du tétrachloroéthylène. Là encore, malgré une température de réaction de 400°C et un rapport molaire $HF/C_2Cl_4$ élevé (5/1), la composition des produits formés se limite au F121 ($CHCl_2-CFCl_2$ : 6,8 %), au F122 ($CHCl_2-CClF_2$ : 10,5 %) et au F123 (82,7 %).

Le brevet US 3 258 500 décrit l'utilisation de chrome massique ou supporté sur alumine pour des réactions de fluoration catalytique en phase gazeuse. En particulier, l'exemple 17 (colonne 14) décrit la fluoration du tétrachloroéthylène. A 400°C avec un rapport molaire $HF/C_2Cl_4$ de 6,2/1, la sélectivité en F123 + F124 + F125 est faible (47,7 %) ; une diminution de la température de réaction (300°C) améliore cette sélectivité (79,7 %) mais la distribution est alors déplacée vers les produits moins fluorés (F123 et F124).

La demande de brevet EP 0 349 298 décrit la synthèse des composés F123 et F124 à partir de pentahalogénoéthanes par fluoration catalytique en phase gazeuse sur un catalyseur composé d'un métal choisi parmi le chrome, le cobalt, le nickel et le manganèse, déposé sur alumine. Ce document met l'accent, d'une part, sur l'activation à l'acide fluorhydrique poussée du catalyseur (au moins 90 % du support sous forme de $AlF_3$ après activation) et, d'autre part, la formation minimisée de F125 durant la réaction. Ainsi, dans l'exemple 6 qui décrit la fluoration du F122 à 350°C et avec un long temps de contact (30 secondes), la sélectivité en F125 n'est que de 1,1 % et la sélectivité cumulée (F123 + F124 + F125) est seulement de 71,5 %. Dans l'exemple 5 qui décrit la fluoration du F123 en phase gazeuse sur un catalyseur $NiCl_2/Al_2O_3$ à 400°C, avec un temps de contact de 30 secondes et un rapport molaire HF/F123 de 4, la sélectivité en F125 est seulement de 7,5 %.

Un procédé pour produire des hydrocarbures aliphatiques fluorés, basé sur la fluoration par l'acide fluorhydrique en phase gazeuse d'hydrocarbures aliphatiques halogénés contenant au moins un atome d'halogène autre que le fluor, fait l'objet du brevet US 3 755 477 où le catalyseur est un oxyde chrome massique traité à la vapeur avant calcination et activation à l'acide fluorhydrique. L'exemple 25 utilise un tel catalyseur pour la fluoration du F123 à 390°C avec un rapport molaire HF/F123 élevé (9,5/1) ; les sélectivités en F125 et F124 sont respectivement de 67 et 21 %, mais on note aussi une sélectivité de 2,5 % en chloropentafluoroéthane (F115) non recyclable.

De l'examen de l'état de la technique, il apparait difficile de synthétiser les deux composés désirés (F124 et F125) avec une bonne sélectivité et une productivité importante par fluoration directe du tétrachloroéthylène ou du F122. Au départ du tétrachloroéthylène et malgré des temps de contact, des températures et des rapports molaires élevés, il semble difficile d'obtenir le F124 et plus spécialement le F125 avec de bons rendements. La synthèse de ces deux composés est plus facile à partir du F122, mais on est confronté dans ce cas à un problème de sélectivité (formation importante de sous-produits).

Quant au brevet US 3 755 477, il montre qu'à partir du F123 la synthèse du F125 requiert un rapport molaire (9,5) et une température (390°C) élevés conduisant concomitamment à la formation non négligeable de F115 indésirable.

Vu l'intérêt des composés F124 et F125 comme substituts aux CFC, leur fabrication industrielle nécessite un procédé particulièrement performant, c'est-à-dire permettant d'obtenir :
- une très grande sélectivité en F124 + F125
- une productivité élevée en F124 et/ou F125
- une grande souplesse pour orienter à volonté la fabrication vers la production majoritaire de F124 ou vers celle de F125, tout en minimisant la formation de sous-produits.

Un tel procédé a maintenant été trouvé qui consiste à fluorer en phase gazeuse au moins un pentahalo-génoéthane de formule $C_2HX_{2-n}F_{3+n}$ dans laquelle X désigne un atome de chlore ou de brome et n le nombre 0 ou 1, au moyen d'acide fluorhydrique sur un catalyseur constitué d'une quantité catalytique de chrome à un degré d'oxydation égal ou supérieur à 3 et d'un charbon actif comme support. Un tel catalyseur permet en effet d'obtenir une sélectivité en F124 + F125 d'environ 95 % ou plus, et de minimiser en même temps la formation de F124a (1-chloro-1,1,2,2-tétrafluoroéthane).

Le pentahalogénoéthane de départ est de préférence le F123, seul ou en mélange avec du F123a (1,2-dichloro-1,1,2-trifluoroéthane). Cependant, pour !a fabrication préférentielle du F125, on peut aussi utiliser comme produit de départ le F124 lui-même, son isomère F124a, le F123b (1,1-dichloro-1,2,2-trifluoroéthane) ou un mélange de ces composés. D'autre part, certains produits de la réaction (par exemple F122, $C_2Cl_4$, CFCl = $CCl_2$, $CF_2 = CCl_2$ et F124a) peuvent être recyclés au réacteur de fluoration. On ne sortirait donc pas du cadre de la présente invention en alimentant le réacteur avec un mélange contenant en moles au moins 50 % de F123 et/ou F124, de 0 à 5 % de composés sous-fluorés et de 0 à 50 % de F123a, F123b et/ou F124a.

Bien qu'on préfère partir des pentahalogénoéthanes chlorés $C_2HCl_{2-n}F_{3+n}$, le procédé selon l'invention peut s'appliquer à leurs homologues bromés tels que, par exemple, le 1-bromo-1-chloro-2,2,2-trifluoroéthane ($CF_3CHBrCl$), le 1,1-dibromo-2,2,2-trifluoroéthane ($CF_3CHBr_2$), le 1-bromo-2-chloro-1,1,2-trifluoroéthane ($CF_2BrCHClF$) ou 1,1-dibromo-1,2,2-trifluoroéthane ($CFBr_2CHF_2$).

Le catalyseur à utiliser conformément à la présente invention peut être préparé par imprégnation d'un charbon actif avec une solution d'un composé à base de chrome. L'espèce active à base de chrome peut se présenter sous forme d'oxydes, hydroxydes, halogénures (à l'exception de l'iode), oxyhalogénures, nitrates, sulfates ou autres composés du chrome, le composé préféré étant l'oxyde de chrome III.

La teneur du catalyseur en espèce active, exprimée en chrome métal, peut aller de 0,1 à 50 % en poids et est de préférence comprise entre 1 et 20 %.

Un catalyseur plus particulièrement préféré est un catalyseur à base d'oxyde de chrome sur un charbon actif présentant une grande surface spécifique totale (supérieure à 800 m²/g). Ce type de catalyseur peut être obtenu, comme indiqué dans le brevet EP 0 055 659, par imprégnation du charbon actif au moyen d'une solution aqueuse de trioxyde de chrome, puis séchage à une température comprise entre 100 et 300°C, de préférence entre environ 150 et 200°C. Dans le catalyseur final, la majeure partie du trioxyde de chrome est réduite à l'état de sesquioxyde $Cr_2O_3$ par le charbon actif lui-même.

L'activation du catalyseur sec est spécifique et doit être adaptée à l'entité catalytique déposée sur le charbon actif support. De manière générale, les catalyseurs non activés ne contenant pas d'oxygène doivent être fluorés en présence d'une source d'oxygène (par exemple, l'air) ou subir un traitement thermique également en présence d'une source d'oxygène. Par contre, un catalyseur dont l'entité catalytique est un oxyde de chrome peut, éventuellement après séchage poussé sous azote (environ 400°C), être directement fluoré avec de l'acide fluorhydrique dilué ou non avec de l'azote. Pour préserver l'activité du catalyseur, il est avantageux de contrôler l'exothermicité de son activation et d'éviter de le porter à une température trop élevée (600-700°C). Il est donc recommandé de commencer la fluoration à basse température, puis, après les "vagues" d'exothermicité, d'augmenter progressivement la température pour atteindre 350 à 450°C en fin d'activation.

Conformément au procédé selon l'invention, la réaction de fluoration du pentahalogénoéthane par HF peut être conduite à une température allant de 250 à 470°C et, plus particulièrement, à une température comprise entre 280 et 410°C. Cependant, si l'on désire orienter la réaction vers la synthèse préférentielle du F124, on travaillera plutôt à une température située dans la partie basse (300-330°C) du domaine précité ; inversement, une température plus élevée favorise la synthèse du F125.

Le temps de contact pour la réaction selon l'invention peut être compris entre 3 et 100 secondes et, plus particulièrement, entre 5 et 30 secondes. Cependant, afin d'obtenir un bon compromis entre le taux de conversion du F123 et des productivités élevées de F124 et/ou F125, la meilleure plage est de 7 à 15 secondes.

Le rapport molaire HF/pentahalogénoéthane peut aller de 1/1 à 20/1 et préférentiellement de 2/1 à 9/1. Là encore, le taux de conversion du F123 et la distribution des produits formés varient avec le rapport molaire choisi, une augmentation du rapport molaire conduisant à une amélioration du taux de conversion du F123 et à un déplacement des produits formés vers des composés plus fluorés (F125). Il faut toutefois noter qu'un rapport molaire faible (inférieur à 2) augmente la formation de produits non recyclables (perhalogénoéthanes et tétrahalogénoéthanes).

La réaction de fluoration selon l'invention peut être conduite dans un réacteur de fluoration en phase gazeuse en lit fixe ou en lit fluide. Les matériaux utilisés pour la construction de l'installation doivent être compatibles avec la présence d'hydracides tels que HCl et HF ; ils peuvent être choisis en "Hastelloy" ou en "Inconel" qui sont résistants aux milieux corrosifs contenant ces hydracides.

La réaction de fluoration selon l'invention peut être menée à la pression atmosphérique ou à une pression supérieure à cette dernière. Pour des raisons pratiques, on opérera généralement dans une zone allant de 0 à 25 bars relatifs.

La mise en oeuvre du procédé selon l'invention permet d'obtenir les deux pentahalogénoéthanes désirés F124 et F125 avec une excellente sélectivité (égale ou supérieure à environ 95 %), la proportion d'isomères a et de produits non recyclables (perhalogénoéthanes et tétrahalogénoéthanes) étant très faible. Le procédé selon l'invention présente également une grande souplesse ; comme le montrent les exemples suivants, le rapport molaire F124/F125 dans le produit obtenu peut varier de 10/1 à 1/10 selon les conditions opératoires. D'autre part, la possibilité d'opérer avec des rapports molaires et des temps de contact faibles permet d'obtenir une bonne productivité, tout en ayant un taux satisfaisant de transformation du F123.

Les produits sous-fluorés formés (F122 : 0-0,2 % et F1111 : 0,1-1,2 %) peuvent être isolés ou recyclés avec les F123 et F123a non transformés. Si on le désire, les composés F124 et F124a peuvent également être recyclés au réacteur pour augmenter la productivité en F125.

Dans les exemples suivants qui illustrent l'invention sans la limiter, les pourcentages indiqués sont des pourcentages molaires et l'acide fluorhydrique utilisé est un produit commercial contenant seulement des traces d'eau.

## EXEMPLE 1: PREPARATION DU CATALYSEUR CrIII/CHARBON

250 ml (103 g) d'un charbon actif végétal préalablement séché à 150°C et présentant les caractéristiques suivantes :
- densité apparente : 0,41
- granulométrie : extrudés de 0,8 mm
- surface BET : 922 m$^2$/g
- surface des pores de 50-250 Å: 20,4 m$^2$/g
- surface des pores de 250-320 Å: 3,2 m$^2$/g

sont imprégnés avec une solution aqueuse de 56 g (0,56 mole) de trioxyde de chrome dans 65 g d'eau.

Le charbon actif absorbe la totalité de la solution aqueuse. Le catalyseur est ensuite séché en lit fluidisé par l'air à 150°C.

L'analyse du catalyseur sec montre que la majeure partie du chrome est à l'état trivalent.

## EXEMPLES 2 A 8: FLUORATION DU F123

Le F123 utilisé comme réactif organique de départ est un produit brut constitué essentiellement de F123 (96,1 %), les autres composés étant le F123a (3,6 %), le F123b (moins de 0,1 %) et le F113 (environ 0,1 %).

Le réacteur utilisé est un tube en Inconel de 250 ml, chauffé au moyen d'un bain fluidisé d'alumine.

Dans ce réacteur, on charge 100 ml du catalyseur obtenu comme décrit à l'exemple 1. Le catalyseur est d'abord séché sous un courant d'air (2 l/h) à 120°C pendant 5 heures, puis à cette même température, on ajoute progressivement de l'acide fluorhydrique à l'air pendant une durée de 3 heures (une mole de HF introduite en 3 heures). Après cette première phase d'activation à basse température, le catalyseur est chauffé sous courant d'azote (1 l/h) jusqu'à 400°C. Lorsque cette température est atteinte, l'azote est remplacé progressivement par de l'acide fluorhydrique pour terminer l'activation avec HF pur (0,5 mole/heure) pendant 8 heures à 400°C.

Sous balayage d'azote (0,5 l/h), la température du catalyseur activé est alors amenée à la valeur choisie pour la réaction de fluoration envisagée. On remplace ensuite le courant d'azote par un courant de HF et de F123 brut, ces réactifs ayant été préalablement mélangés et portés à la température de réaction dans un préchauffeur en Inconel. Les proportions de HF et de F123 et le débit d'alimentation sont réglés en fonction des valeurs choisies pour le rapport molaire HF/F123 et le temps de contact.

Après lavage à l'eau pour éliminer les hydracides et séchage sur chlorure de calcium, les produits issus du réacteur sont analysés en ligne par chromatographie en phase gazeuse. L'appareil utilisé est un chromatographe DELSI IGC 11 muni d'un filament de 200 mA en tungstène et d'une colonne de 5 m de long et de 3,175 mm de diamètre, remplie avec un mélange de UCON LB 550 X (polypropylèneglycol) à 25 % sur SPHEROSIL XOA 200 (support en silice poreuse). Le gaz vecteur est l'hélium (20 ml/min) et la température d'analyse est maintenue constante à 100°C.

Le tableau suivant rassemble les conditions opératoires et les résultats obtenus dans différentes opérations réalisées à pression atmosphérique, sur deux lots différents du même catalyseur : le premier lot pour les opérations 2 à 5 et le second pour les opérations 6 à 8. L'âge du catalyseur correspond à sa durée totale de fonctionnement depuis sa mise en service, c'est-à-dire depuis la première introduction d'un mélange HF + F123 brut (respectivement opérations 2 et 6).

| EXEMPLES | 2 | 3 | 4 | 5 | 6 | 7 | 8 |
|---|---|---|---|---|---|---|---|
| **CONDITIONS OPERATOIRES** | | | | | | | |
| - Température (°C) | 350 | 350 | 300 | 330 | 340 | 340 | 340 |
| - Rapport molaire HF/F123 | 5 | 2,9 | 3 | 2 | 3,3 | 4,3 | 3,3 |
| - Temps de contact (secondes) | 9,7 | 9,7 | 11,2 | 9,7 | 9,9 | 9,9 | 10,3 |
| - Age du catalyseur (heures) | 59 | 65 | 69 | 87 | 60 | 84 | 210 |
| **RESULTATS** | | | | | | | |
| - Taux de transformation du F123 (%) | 90,4 | 83,4 | 49,2 | 66,8 | 78,8 | 83,5 | 78,4 |
| - Sélectivité (%) en : $F124$ ($CF_3CHFCl$) | 17,7 | 22,6 | 87,8 | 43 | 25,9 | 24,9 | 27 |
| $F125$ ($CF_3CHF_2$) | 78,8 | 71,6 | 10 | 52,4 | 70,1 | 72 | 69 |
| $F122$ ($CF_2ClCHCl_2$) | 0 | 0,05 | 0 | 0,1 | 0,05 | 0 | 0 |
| $F123a$ ($CF_2ClCHFCl$) | 0 | 0 | 0 | 0 | 0 | 0 | 0 |
| $F124a$ ($CF_2ClCHF_2$) | 0,2 | 0,3 | 0,8 | 0,2 | 0,3 | 0,2 | 0,3 |
| $F1111$ ($Cl_2C=CFCl$) | 0,1 | 0,2 | 0,1 | 0,1 | 0,3 | 0,1 | 0,2 |
| $F114 + F114a$ ($C_2F_4Cl_2$) | 1,7 | 2,1 | 0,3 | 1,5 | 1,3 | 1,1 | 1 |
| $F115$ ($C_2ClF_5$) | 0,4 | 0,5 | 0,1 | 0,4 | 0,3 | 0,3 | 0,5 |
| $F133a$ ($CF_3CH_2Cl$) | 0,9 | 1,3 | 0,6 | 1,3 | 1,1 | 1 | 0,9 |
| $F1110$ ($Cl_2C=CCl_2$) | 0 | 1,2 | 0 | 0,6 | 0,5 | 0,1 | 0,4 |

La comparaison des exemples 6 et 8 montre que l'activité catalytique se maintient parfaitement dans le temps.

## Revendications

1. Procédé de préparation du 1,1,1,2-tétrafluorochloroéthane et/ou du pentafluoroéthane par fluoration catalytique en phase gazeuse d'au moins un pentahalogénoéthane de formule $C_2HX_{2-n}F_{3+n}$ dans laquelle X désigne un atome de chlore ou de brome et n le nombre 0 ou 1, au moyen d'acide fluorhydrique, caractérisé en ce que l'on utilise un catalyseur constitué d'une quantité catalytique de chrome à un degré d'oxydation égal ou supérieur à 3 et d'un charbon actif comme support.

2. Procédé selon la revendication 1, caractérisé en ce que le chrome est déposé sur le charbon actif sous forme d'oxyde, d'hydroxyde, d'halogénure (iode excepté), d'oxyhalogénure, de nitrate ou de sulfate de chrome.

3. Procédé selon la revendication 2, dans lequel le chrome est sous forme d'oxyde de chrome III.

4. Procédé selon l'une des revendications 1 à 3, dans lequel la teneur du catalyseur en espèce active, exprimée en chrome métal, est comprise entre 0,1 et 50 % en poids, de préférence entre 1 et 20 %.

5. Procédé selon l'une des revendications 1 à 4, dans lequel l'acide fluorhydrique et le(s) pentahalogénoéthane(s) sont mis en contact dans un rapport molaire HF/pentahalogénoéthane(s) compris entre 1/1 et 20/1, pendant un temps compris entre 3 et 100 secondes et à une température comprise entre 250 et 470°C.

6. Procédé selon la revendication 5, dans lequel le rapport molaire est compris entre 2/1 et 9/1, le temps de contact est compris entre 5 et 30 secondes, de préférence entre 7 et 15 secondes, et la température est comprise entre 280 et 410°C.

7. Procédé selon l'une des revendications 1 à 6, dans lequel le pentahalogénoéthane de départ est le 1,1,1-trifluoro-dichloroéthane et/ou le 1,1,1,2-tétrafluoro-chloroéthane.

8. Procédé selon l'une des revendications 1 à 6, dans lequel le produit de départ est un mélange contenant en moles au moins 50 % de 1,1,1-trifluoro-dichloroéthane et/ou 1,1,1,2-tétrafluoro-chloroéthane, de 0 à 5 % de composés sous-fluorés recyclés, et de 0 à 50 % de 1,1,2-trifluoro-1,2-dichloroéthane, 1,2,2-trifluoro-1,1-dichloroéthane et/ou 1,1,2,2-tétrafluoro-chloroéthane.

9. Procédé selon l'une des revendications 1 à 8, dans lequel on travaille à une pression allant de 0 à 25 bars relatifs, de préférence à la pression atmosphérique.


## Claims

1. Process for the preparation of 1,1,1,2-tetrafluorochloroethane and/or of pentafluoroethane by gas phase catalytic fluorination of at least one pentahaloethane of formula $C_2HX_{2-n}F_{3+n}$ in which X denotes a chlorine or bromine atom and n the number 0 or 1, by means of hydrofluoric acid, characterized in that a catalyst is employed consisting of a catalytic quantity of chromium in an oxidation state equal to or higher than 3 and of an activated charcoal as support.

2. Process according to Claim 1, characterized in that the chromium is deposited onto the activated charcoal in the form of chromium oxide, hydroxide, halide (iodine excepted), oxyhalide, nitrate or sulphate.

3. Process according to Claim 2, in which the chromium is in the form of chromium(III) oxide.

4. Process according to one of Claims 1 to 3, in which the active species content of the catalyst, expressed as chromium metal, is between 0.1 and 50 % by weight, preferably between 1 and 20 %.

5. Process according to one of Claims 1 to 4, in which the hydrofluoric acid and the pentahaloethane(s) are brought into contact in an HF/pentahaloethane(s) molar ratio of between 1/1 and 20/1 for a time of between 3 and 100 seconds and at a temperature of between 250 and 470°C.

6. Process according to Claim 5, in which the molar ratio is between 2/1 and 9/1, the contact time is between 5 and 30 seconds, preferably between 7 and 15 seconds, and the temperature is between 280 and 410°C.

7. Process according to one of Claims 1 to 6, in which the starting pentahaloethane is 1,1,1-trifluorodichloroethane and/or 1,1,1,2-tetrafluorochloroethane.

8. Process according to one of Claims 1 to 6, in which the starting material is a mixture containing, on a molar basis, at least 50 % of 1,1,1-trifluorodichloroethane and/or 1,1,1,2-tetrafluorochloroethane, from 0 to 5 % of recycled underfluorinated compounds and from 0 to 50 % of 1,1,2-trifluoro-1,2-dichloroethane, 1,2,2-trifluoro-1,1-dichloroethane and/or 1,1,2,2-tetrafluorochloroethane.

9. Process according to one of Claims 1 to 8, in which the work is done at a pressure ranging from 0 to 25 bars gauge, preferably at atmospheric pressure.

**Patentansprüche**

1. Verfahren zur Herstellung von 1,1,1,2-Tetrafluorchlorethan und/oder Pentafluorethan durch katalytische Gasphasen-Fluorierung von mindestens einem Pentahalogenethan der Formel $C_2HX_{2-n}F_{3+n}$, in der X ein Chlor- oder Bromatom und n die Zahl 0 oder 1 bezeichnet, mit Fluorwasserstoffsäure, dadurch gekennzeichnet, daß man einen Katalysator verwendet, der aus einer katalytischen Menge Chrom der Oxydationsstufe gleich oder größer 3 und einem Träger aus Aktivkohle besteht.

2. Verfahren nach Anspruch 1, dadurch gekennzeichnet, daß das Chrom auf die Aktivkohle als Chromoxid, -hydroxid, -halogenid (mit Ausnahme des Jodids), -oxyhalogenid, -nitrat oder -sulfat aufgebracht ist.

3. Verfahren nach Anspruch 2, dadurch gekennzeichnet, daß das Chrom als Chrom(III)-oxid vorliegt.

4. Verfahren nach einem der Ansprüche 1 bis 3, dadurch gekennzeichnet, daß der aktive Anteil des Katalysators, berechnet als metallisches Chrom, zwischen 0,1 und 50 Gew.%, bevorzugt zwischen 1 und 20, liegt.

5. Verfahren nach einem der Ansprüche 1 bis 4, dadurch gekennzeichnet, daß die Fluorwasserstoffsäure und das (die) Pentahalogenethan(e) in einem HF/Pentahalogenethan(e)-Molverhältnis zwischen 1/1 und 20/1, während einer Zeitdauer zwischen 3 und 100 Sekunden und bei einer Temperatur zwischen 250 und 470°C, zusammengebracht werden.

6. Verfahren nach Anspruch 5, dadurch gekennzeichnet, daB das Molverhältnis zwischen 2/1 und 9/1, die Kontaktzeit zwischen 5 und 30 Sekunden, bevorzugt zwischen 7 und 15 Sekunden, und die Temperatur zwischen 280 und 410°C liegen.

7. Verfahren nach einem der Ansprüche 1 bis 6, dadurch gekennzeichnet, daß das Ausgangspentahalogenethan 1,1,1,-Trifluordichlorethan und/oder 1,1,1,2-Tetrafluorchlorethan ist.

8. Verfahren nach einem der Ansprüche 1 bis 6, dadurch gekennzeichnet, daß das Edukt eine Mischung ist, die in Mol mindestens 50 % 1,1,1-Trifluordichlorethan und/oder 1,1,1,2-Tetrafluorchlorethan, 0 bis 5 % recyclierte unterfluorierte Bestandteile, und 0 bis 50 % 1,1,2-Trifluor-1,2-dichlorethan, 1,2,2-Trifluor-1,1-dichlorethan und/oder 1,1,2,2-Tetrafluorchlorethan enthält.

9. Verfahren nach einem der Ansprüche 1 bis 8, dadurch gekennzeichnet, daß man bei einem relativen Druck zwischen 0 und 25 bar, bevorzugt bei Atmosphärendruck, arbeitet.